# FASCICULE DE BREVET EUROPEEN

(11) **EP 1 326 636 B1**
(45) Date de publication et mention de la délivrance du brevet: **03.01.2007**
(21) Numéro de dépôt: 01976380.4
(22) Date de dépôt: 08.10.2001
(51) Int. Cl.: A61K 39/21

(54) **COMPOSITION VACCINALE**
IMPFSTOFFZUSAMMESETZUNG
VACCINE COMPOSITION

(30) Priorité: 06.10.2000 FR 0012808
(43) Date de publication de la demande: 16.07.2003
(73) Titulaire: Sanofi Pasteur, 69367 Lyon Cedex 07 (FR)
(72) Inventeur: HAENSLER, Jean, F-69290 Saint Genis les Ollières (FR); HURPIN, Christian, Marcel, F-69280 St Didier au Mont d'Or (FR)
(74) Mandataire: Kerneis, Daniéle
(86) Numéro de dépôt international: PCT/FR2001/003098
(87) Numéro de publication internationale: WO 2002/028428

(56) Documents cités:
- WO-A-00/01345
- WO-A-00/15256
- WO-A-00/50006
- WO-A-01/15726
- FARHOOD, HASSAN ET AL: "Codelivery to mammalian cells of a transcriptional factor with cis-acting element using cationic liposomes" ANALYTICAL BIOCHEMISTRY (1995), 225(1), 89-93 , XP001126233

## Description

L'invention est relative au domaine des compositions vaccinales. Plus particulièrement, l'invention concerne une composition vaccinale adjuvée.

On connaît dans l'art antérieur de nombreux adjuvants susceptibles d'être utilisés dans le domaine des vaccins afin d'améliorer la réponse immunitaire induite lors de leur administration.
Ainsi, par exemple, la demande de brevet WO96/14831 décrit l'utilisation d'adjuvants constitués par des composés amphipathiques comprenant un groupement lipophile dérivé d'un stérol lié à un groupement cationique, tels que le 3 β-[N-(N',N'-diméthylaminoéthane)-carbamoyl] cholestérol, encore appelé DC chol.
La demande de brevet WO98/18810, quant à elle, décrit des nucléotides dont la séquence nucléotidique possède des motifs particuliers (un dinucléotide CG encadré par l'Adénine, la Guanine ou la Thymine d'un côté et la Cytosine ou la Thymine de l'autre côté), pour leur utilisation en tant qu'immunostimulants, notamment lors de l'administration de vaccins.
Ces demandes ne sont que des exemples parmi l'importante littérature relative à ce sujet.
Or, bien que de nombreuses substances aient été décrites dans l'art antérieur en relation avec leurs propriétés d'adjuvants vaccinaux, on cherche toujours à améliorer la qualité et l'efficacité des vaccins grâce, notamment, à l'utilisation de nouveaux adjuvants qui permettraient, soit de diminuer la quantité d'antigènes présents dans le vaccin pour obtenir une réponse immunitaire satisfaisante, soit d'orienter la réponse immunitaire dans la direction souhaitée en fonction, par exemple, de la maladie concernée, de la voie d'administration choisie ou de l'effet recherché (prévention ou traitement).
Une des difficultés est liée au fait que, même si les réponses du système immunitaire sont de mieux en mieux connues, il reste très difficile, voire impossible, de les anticiper, et que très souvent, la combinaison de 2 adjuvants conduit à un résultat décevant, soit parce que la toxicité est alors trop grande, soit parce que chacun des adjuvants, actif isolément, semble avoir un effet inhibiteur ou neutralisant sur l'adjuvant qui lui est associé.
Le but de la présente invention est donc de proposer une nouvelle composition vaccinale dont l'immunogénicité est améliorée par rapport à l'art antérieur, i.e. que la réponse immunitaire induite consécutivement à son administration est augmentée par rapport à l'art antérieur.

Pour atteindre ce but, l'invention a pour objet l'utilisation d'une composition comprenant au moins un antigène, un lipide cationique et un oligonucléotide pour la fabrication d'un vaccin devant être administré par voie muqueuse.

Selon une caractéristique de l'invention, ledit lipide cationique est du DCchol.

Selon une caractéristique particulière de l'invention, ledit antigène est un antigène du virus VIH.

La présente invention sera mieux comprise à la lecture de la description détaillée qui va suivre.

Par composition vaccinale au sens de la présente invention, on entend une composition susceptible d'être administrée à l'homme ou à l'animal afin d'induire une réponse du système immunitaire; cette réponse du système immunitaire pouvant se traduire par une production d'anticorps ou simplement, par une activation de certaines cellules, notamment les cellules présentatrices d'antigènes, les lymphocytes T, les lymphocytes B. La composition vaccinale peut être une composition à visée prophylactique ou à visée thérapeutique, ou encore les deux.

La composition vaccinale est administrée par voie muqueuse et permet, dans le cas des microorganismes ayant une porte d'entrée muqueuse, d'induire une réponse immunitaire de type muqueuse, avec production d'immunoglobulines A spécifiques.
Ainsi, il peut être intéressant de rechercher ce type de réponse dans la vaccination contre les virus à porte d'entrée respiratoire (Virus Synctial Respiratoire, virus de la grippe, parainfluenza virus, etc...), à porte d'entrée digestive (virus de la polio, rotavirus,...) ou encore à porte d'entrée vaginale ou rectale ( VIH, hépatite B,....)
De la même façon, une réponse immunitaire de type muqueux est recherchée dans les affections bactériennes provoquées par exemple par Chlamydia, Neisseria gonorrheae, Streptococcus pneumoniae, Haemophilus influenzae, Moraxella catarrhalis.
Par contre, dans d'autres cas, on souhaite plutôt induire une réponse de type TH1 avec production de cellules cytotoxiques; ceci est notamment le cas pour les virus non cytopathiques tels que les cytomegalovirus, les microorganismes intracellulaires (Bacille de Koch, des parasites comme Falciparum ou Leishmania, des bactéries telles que Listeria, Legionella, Yersinia enterolitica) ou d'autres comme les Spirochètes.
Dans certains cas encore, on peut souhaiter induire plusieurs types de réponse; c'est notamment le cas pour la grippe ou le Sida. Dans de tels cas, la composition selon l'invention est d'un intérêt tout particulier, car elle permet, alors, d'obtenir différents types de réponse du système immunitaire.

Par antigène au sens de la présente invention, on entend tout antigène susceptible d'être utilisé dans un vaccin, qu'il s'agisse de germe entier ou de sous-unité et quelle que soit sa nature: peptide, protéine, glycoprotéine, polysaccharide, glycolipide, lipopeptide... etc. Il peut s'agir d'antigènes viraux, bactériens ou autres; le terme antigène comprend également les polynucléotides dont les séquences sont choisies pour coder les antigènes que l'on souhaite voir exprimés par les individus auxquels sont administrés les polynucléotides, dans le cas de la technique d'immunisation que l'on appelle immunisation ADN. Il peut également s'agir d'un ensemble d'antigènes, notamment dans le cas d'une composition vaccinale multivalente qui comprend des antigènes susceptibles de protéger contre plusieurs maladies, ou dans le cas d'une composition qui comprend plusieurs antigènes différents pour protéger contre une seule maladie, ainsi que cela est le cas pour certains vaccins contre la coqueluche ou la grippe par exemple.

Par lipide cationique au sens de la présente invention, on entend un composé formé d'une partie grasse (par exemple une ou plusieurs chaines hydrophobes ou un noyau stérol) et d'une tête polaire chargée positivement au pH physiologique.En particulier, il peut s'agir d'un composé comprenant un groupement lipophile dérivé d'un stérol lié à un groupement cationique et notamment un dérivé de cholestérol relié à un ammonium quaternaire ou à une amine protonable par une liaison carbamoyle. Une telle liaison présente en effet l'avantage d'être hydrolysable dans la cellule.De tels composés peuvent se trouver sous forme basique, sous forme de sel, ou encore, et c'est le cas le plus fréquent, à la fois sous les 2 formes en équilibre dans un mélange, le déplacement de l'équilibre vers l'une ou l'autre forme dépendant de la composition du mélange et notamment de son pH. Un des lipides cationiques particulièrement intéressant aux fins de l'invention est le DCchol qui peut être obtenu à partir de cholesteryl chloroformate et de N, N-dimethylethylenediamine, selon la méthode décrite dans le brevet US 5283185 ou de façon préférée, selon la méthode décrite à l'exemple 8 de la demande de brevet WO 96/40067. Il est possible également d'utiliser un produit obtenu par réaction de cholestéryl chloroformate et de N,N,N triméthyléthylènediamine.

Par oligonucléotide au sens de la présente invention, on comprend un oligonucléotide simple brin ayant de 6 à 100 nucléotides, de préférence de 6 à 30 nucléotides. Il peut s'agir d'oligoribonucléotide, ou d'oligodesoxyribonucléotide. On utilise notamment des oligonucléotides comprenant au moins une séquence dinucléotidique Cytosine, Guanine, dans laquelle ni la Cytosine ni la Guanine ne sont méthylées. Tout autre oligonucléotide connu pour être, par sa nature même, immunostimulant, peut également convenir aux fins de l'invention. On a obtenu de particulièrement bons résultats en utilisant un oligonucléotide dont la séquence est décrite dans la demande de brevet WO96/02555 sous SEQ ID N° 15, qui est reprise ci-après: 5' GAGAACGCTCGACCTTCGAT 3'.
Les oligonucléotides convenant aux fins de l'invention peuvent se présenter sous forme de phosphodiester, ou sous toute autre forme étudiée afin de les améliorer, notamment en terme de stabilité; ainsi, il est possible d'utiliser des oligonucléotides se présentant sous forme de phosphorothioates ou d'hybrides phosphodiester/phosphorothioates. Bien qu'il soit possible d'utiliser des oligonucléotides provenant de sources d'acides nucléiques existantes tel que l'ADN génomique ou le cADN, on préfère utiliser des oligonucléotides de synthèse. Ainsi, il est possible d'élaborer des oligonucléotides sur support solide en utilisant la méthode β-cyano éthyl phosphoramidite (Beaucage, S.L. and Caruthers, M.H. Tetrahedron Letters 22, 1859 - 1862 (1981)) pour l'assemblage 3'-5'.
Les oligonucléotides phosphorothioatés ont un des atomes d'Oxygène composant le groupement Phosphate qui est remplacé par un atome de Soufre. Leur synthèse peut être effectuée comme précédemment décrit, sauf à remplacer la solution iode/eau/pyridine tétrahydrofurane qui est utilisée lors de l'étape d'oxydation nécessaire à la synthèse des liaisons phosphodiester par une solution TETD (tétraethylthiuram disulfide) apportant les ions sulfates permettant de produire le groupement phosphorothioate.
On peut également envisager d'autres modifications des liaisons phosphodiesters, des bases ou des sucres, pour modifier les propriétés des oligonucléotides utilisés, et notamment pour accroître leur stabilité.

Par réponse immunitaire de type Th1 au sens de la présente invention, on entend une réponse immunitaire spécifique de l'antigène caractérisée en ce qu'elle entraine une production orientée de cytokines, principalement l'Interféron γ et l'IL2, et une production massive de certaines sous-classes d'anticorps (i.e. IgG2a chez la souris).
On peut également observer une production de cellules T cytotoxiques.

Par réponse immunitaire de type Th2, on entend une réponse immunitaire qui se traduit par une production majoritaire d'IL4 et d'IL5, ainsi que par une production massive de certaines autres sous-classes d'anticorps ( i.e IgG1 chez la souris).

Lorsqu'on veut étudier le type de réponse immunitaire induite par une composition vaccinale, on peut effectuer des dosages comparatifs des IgG1 et des IgG2a spécifiques produites lors de l'administration de la composition vaccinale étudiée à des souris; une réponse de type Th1 se traduit par une plus forte production d' IgG2a spécifiques conduisant à une valeur faible du rapport IgG1/ IgG2a, alors qu'une réponse de type Th2 se traduit par une plus forte production d' IgG1 spécifiques conduisant à une valeur forte du rapport IgG1/IgG2a.
Alternativement, le dosage des cytokines produites permet également, lors de tests in vitro ou sur animaux, d'apprécier l'orientation de la réponse immunitaire; on peut notamment effectuer le rapport IL5/INFγ; une réponse de type Th1 se traduisant par une valeur faible de ce rapport alors qu'une réponse de type Th2 se traduit plutôt par une valeur élevée de ce rapport.
Il est également possible d'observer la quantité d' IgA dont la production traduit une orientation de la réponse immunitaire vers le type Th2.
Or, selon les cibles vaccinales, i.e. les maladies contre lesquelles sont destinées les compositions vaccinales, il peut être souhaitable de pouvoir orienter la réponse immunitaire.

Les exemples qui suivent illustrent, de façon non limitative, des modes de réalisation de l'invention.

### Exemple 1

On a utilisé du chorhydrate de DC-Chol (obtenu selon le mode de préparartion décrit à l'exemple 8 de la demande de brevet WO 96/40067) que l'on a mis en suspension à 20 mg/ml dans du tampon TRIS-NaCl (20 mM TRIS, 150 mM NaCl, pH 6.8). Après 8 heures sous agitation à 35-40°C sous argon, la suspension a été microfluidisée à l'aide d'un microfluidiseur M-110S de chez Microfluidics (10 cycles à 500 kPa), afin de générer une suspension homogène de DC-Chol, que l'on a filtrée sur un filtre Millex 0.45 µm .

### Exemple 2

On a préparé des oligonucléotides grâce à un automate synthétiseur fourni par Applied Biosystems qui met en oeuvre la méthode chimique standard au phosphoramidite et qui comporte à chaque cycle une étape d'oxydation.
Cette étape d'oxydation a été réalisée au moyen d'une solution iode/eau/tétrahydrofurane/acétonitrile pour obtenir une liaison phosphodiester et au moyen d'une solution tétraéthylthiuram/acétonitrile pour obtenir une liaison phosphorothioate.
On a ainsi préparé un oligonucléotide 3 Db(S) dont la séquence est reproduite dans la demande de brevet WO96/02555 sous SEQ ID NO 15 et qui comporte des liaisons phosphorothioate sur toute sa longueur.
On a préparé également un oligonucléotide MGC (S) dont la séquence est reproduite dans la demande de brevet WO00/15256 à SEQ ID NO 2, qui comporte à la fois des liaisons phosphodiester et des liaisons phosphorothioate. Les liaisons phosphorothiate sont situées à chaque extrémité ; il y a 2 liaisons phosphorothioate en 3' et 5 liaisons phosphorothioate en 5'. Cet oligonucléotide ne possède pas de séquence CG et est utilisé comme contrôle négatif.

### Exemple 3

On a préparé des compositions vaccinales contre le virus de l'immunodéficience humaine de type 1 (VIH-1) dans lesquelles l'antigène est la glycoprotéine d'enveloppe gp160 MN/LAI-2. Cet antigène contient la portion gp120 de l'isolat VIH-1 MN et la portion gp41 de l'isolat VIH-1 LAI. La gp41 a été délétée de son site de clivage avec la gp120 et de sa partie transmembranaire de façon à obtenir une glycoprotéine non clivée et essentiellement sécrétée. L'antigène est produit à partir de la lignée cellulaire de hamster BHK-21 infectée par le virus recombinant de la vaccine VVTG.9150 dérivé de la précédente construction VVTG.1163 (Kieny, M.-P. et al, 1988, Protein Eng, 2(3) : 219-255), puis est purifié par chromatographie d'échange d'ions suivie d'une chromatographie d'immunoaffinité.

Les doses vaccinantes de 20µl répondaient à l'une des formulations suivantes:
- 25 µg de gp160 uniquement,
- 25 µg de gp160 + 50 µg d'oligonucléotide 3Db(S) préparé à l'exemple 2,
- 25 µg de gp160 + 50 µg d'oligonucléotide MGC préparé à l'exemple 2 + 200 µg de DCchol préparé à l'exemple 1,
- 25 µg de gp160 + 50 µg d'oligonucléotide 3Db(S) préparé à l'exemple 2 + 200 µg de DCchol préparé à l'exemple 1.
On a injecté les doses vaccinantes préparées à 4 groupes de 6 souris (1 formulation par groupe) par voie rectale, sous anesthésie, à raison de 4 injections séparées chacune de 2 semaines ( soient J1, J15, J29 et J44).
A J57, on a prélevé du sérum, on a recueilli les fécès et on a procédé à des lavages rectaux afin d'effectuer les dosages suivants:
- dosage par ELISA des IgG anti-gp 160 dans le sérum,
- dosage par ELISA des IgA et des IgG totales ainsi que des IgA et des IgG spécifiques anti-gp160 dans les lavages rectaux,
- dosage par ELISA des IgA et des IgG totales ainsi que des IgA et des IgG spécifiques anti-gp160 dans les fécès.

La composition vaccinale contenant l'oligonucléotide MGC a été considérée comme un contrôle négatif par rapport à l'oligonucléotide 3Db(S). En effet, l'oligonucléotide MGC s'était révélé ne pas être immunostimulant dans des expériences précédentes.

Les résultats obtenus sont récapitulés dans les tableaux ci-après; seules les moyennes par groupe de souris ayant reçu la même composition vaccinale sont indiquées.

**Tableau 1: Dosage des IgG spécifiques dans le sérum:**

| | IgG anti-gp 160 en µg/ml |
|---|---|
| 25 µg gp160 | 60,55 |
| 25 µg gp160 + 50 µg 3Db(S) | 46,97 |
| 25 µg gp160 + DCchol 200µg + 50µg MGC | 47,85 |
| 25 µg gp160 + DCchol 200µg +50µg 3Db(S) | 645,26 |

Ces résultats montrent la synergie exercée par les 2 adjuvants pour la production d'IgG vis-à-vis de l'antigène gp160, lors d'une administration par voie muqueuse.

**Tableau 2: Dosage des IgA et des IgG dans les lavages rectaux:**

| | IgA spéc./IgA tot en % | IgG spéc./IgG tot. en % |
|---|---|---|
| 25 µg gp160 | 0,15 | 3,68 |
| 25 µg gp160 + 50 µg 3Db(S) | 0,91 | 2,46 |
| 25 µg gp160 + DCchol 200µg + 50µg MGC | 0,68 | 1,07 |
| 25 µg gp160 + DCchol 200µg +50µg 3Db(S) | 1,53 | 12,43 |

**Tableau 3: Dosage des IgA et des IgG dans les fécès:**

| | IgA spéc./IgA tot. x 10⁴ | IgG spéc./IgG tot. en % |
|---|---|---|
| 25 µg gp160 | 2,44 | 0,00 |
| 25 µg gp160 + 50 µg 3Db(S) | 18,05 | 1,33 |
| 25 µg gp160 + DCchol 200µg + 50µg MGC | 43,38 | 0,00 |
| 25 µg gp160 + DCchol 200µg +50µg 3Db(S) | 104,79 | 3,03 |

Ces résultats montrent l'effet synergique obtenu grâce à l'objet de la présente invention, vis-à-vis de la production locale d'immunoglobulines G et d'immunoglobulines A spécifiques.

Cette capacité à stimuler localement la production d'IgA spécifiques est particulièrement recherchée dans certaines applications vaccinales, et confirme l'intérêt de l'objet de la présente invention.

## Revendications

1. Utilisation d'une composition comprenant au moins un antigène, un lipide cationique et un oligonucléotide pour la fabrication d'un vaccin devant être administré par voie muqueuse.

2. Utilisation selon la revendication précédente, **caractérisée en ce que** ledit lipide cationique est du DCchol.

3. Utilisation selon une des revendications précédentes, **caractérisée en ce que** ledit antigène est un antigène du virus VIH.

## Claims

1. Use of a composition comprising at least one antigen, one cationic lipid and one oligonucleotide, for the manufacture of a vaccine which must be administered mucosally.

2. Use according to the preceding claim, **characterized in that** said cationic lipid is DCchol.

3. Use according to either of the preceding claims, **characterized in that** said antigen is an antigen of the HIV virus.

## Patentansprüche

1. Verwendung einer Zusammensetzung, die mindestens ein Antigen, ein kationisches Lipid und ein Oligonucleotid einschließt, zur Herstellung eines Impfstoffs, der über die Schleimhaut verabreicht werden muss.

2. Verwendung nach dem vorhergehenden Anspruch, **dadurch gekennzeichnet, dass** das kationische Lipid DC-Chol ist.

3. Verwendung nach einem der vorhergehenden Ansprüche, **dadurch gekennzeichnet, dass** das Antigen ein Antigen des HIV-Virus ist.
